# FASCICULE DE BREVET EUROPEEN

(11) **EP 3 528 835 B1**
(45) Date de publication et mention de la délivrance du brevet: **03.03.2021**
(21) Numéro de dépôt: 17797395.5
(22) Date de dépôt: 20.10.2017
(51) Int. Cl.: A61K 38/21, A61K 45/06, A61K 31/351, A61K 31/352, A61K 31/4375, A61K 31/7056, A61P 31/14

(54) **COMPOSITIONS ANTIVIRALES POUR LE TRAITEMENT DES INFECTIONS LIEES AUX CORONAVIRUS**
ANTIVIRALE ZUSAMMENSETZUNGEN ZUR BEHANDLUNG VON INFEKTIONEN IM ZUSAMMENHANG MIT CORONAVIREN
ANTIVIRAL COMPOSITIONS FOR THE TREATMENT OF INFECTIONS LINKED TO CORONAVIRUSES

(30) Priorité: 21.10.2016 FR 1660223
(43) Date de publication de la demande: 28.08.2019
(73) Titulaire: Université Claude Bernard Lyon 1, 69100 Villeurbanne (FR); Institut National de la Santé et de la Recherche Médicale, 75013 Paris (FR); Centre National de la Recherche Scientifique, 75016 Paris (FR)
(72) Inventeur: ROSA-CALATRAVA, Manuel, 69003 Lyon (FR); TERRIER, Olivier, 69008 Lyon (FR); PROUST, Anaïs, 69006 Lyon (FR); MOULES, Vincent, 01600 Trévoux (FR)
(74) Mandataire: Regimbeau
(86) Numéro de dépôt international: PCT/FR2017/052889
(87) Numéro de publication internationale: WO 2018/073549

(56) Documents cités:
- WO-A1-2004/037237
- WO-A1-2015/157223
- CN-A- 1 089 851
- US-A1- 2011 218 241
- DARRYL FALZARANO ET AL: "Treatment with interferon-[alpha]2b and ribavirin improves outcome in MERS-CoV-infected rhesus macaques", NATURE MEDICINE, vol. 19, no. 10, 8 septembre 2013 (2013-09-08), pages 1313-1317, XP055369198, ISSN: 1078-8956, DOI: 10.1038/nm.3362

## Description

La présente invention se rapporte à des compositions pour leur utilisation dans le traitement des infections virales liées au coronavirus MERS-CoV (Middle East Respiratory Syndrome), chez l'homme et chez les animaux.

Le coronavirus MERS-CoV, est un nouveau virus émergent identifié en 2012 en Arabie Saoudite, responsable de syndrome respiratoire aigu grave et de défaillance rénale. Depuis son identification, ce virus a été responsable de plus de 1806 cas d'infection dans 26 pays, principalement au Moyen Orient. Il est responsable de 643 décès soit près de 35,6 % de mortalité d'après l'Organisation Mondiale de la Santé (Source OMS, 28 Septembre 2016).

Le MERS-CoV appartient à l'ordre des *Nidovirales,* à la famille des *Coronaviridae,* et au genre *Betacoronavirus.*

Les coronavirus sont des virus enveloppés, possédant une capside présentant une symétrie hélicoïdale. Ils possèdent un génome ARN simple brin, de sens positif, et sont capables d'infecter les cellules des oiseaux et des mammifères. Les virus membres de cette très large famille sont connus pour être des agents causatifs du rhume (par exemple les virus hCoV et OC43), des bronchiolites (par exemple le virus NL63) ou encore de certaines formes de pneumonies sévères comme celles observées lors de l'épidémie de SARS (tel que le Severe Acute Respiratory Syndrome Coronavirus, SARS-CoV).

Malgré leur appartenance à une même famille virale, d'importantes différences existent entre les différents coronavirus, à la fois au niveau génétique et structural, mais aussi en termes de biologie et de sensibilité aux molécules antivirales, (voir pour référence Dijkman & van der Hoek 2009 ; de Wit *et al.,* 2016)

Bien que la majorité des cas de MERS-CoV chez l'homme soient attribuables à une transmission interhumaine, le chameau apparaît être un hôte animal infecté intermédiaire permanent du MERS-CoV et constitue ainsi la source animale principale de l'infection chez l'homme.

Il n'existe à l'heure actuelle aucune solution prophylactique ou thérapeutique pour traiter efficacement ce pathogène viral respiratoire épidémique au potentiel pandémique.

Plusieurs pistes thérapeutiques ont été récemment explorées : utilisation de la ribavirine, d'interféron, ou encore d'acide mycophénolique. Malheureusement, la plupart de ces composés n'ont pas montré d'efficacité suffisante, lorsque utilisés chez les patients infectés (Al-Tawfiq *et al.,* 2014) ou dans le cadre d'un traitement prophylactique (de Wit *et al.,* 2016.).

Une première stratégie pour l'identification de composés actifs contre le MERS-CoV a été de tester, parmi les nombreuses molécules antivirales connues, celles utilisées pour combattre le SARS-CoV. Ainsi, des inhibiteurs de réplication virale, tels que les inhibiteurs de protéase, les inhibiteurs de l'hélicase, et les inhibiteurs d'entrée du virus dans les cellules cibles ont été testés *in vitro* (voir pour revue Adedeji & Sarafianos, 2014, et Kilianski & Baker, 2014).

Dyall et collaborateurs (Dyall *et al.,* 2014) ont testé différentes catégories de médicaments dans le but d'identifier des agents antiviraux actifs sur les coronavirus SARS et/ou MERS-COV. Parmi les différentes classes d'agents testés, il a été montré que certains agents anti-inflammatoires inhibaient la prolifération du SARS-CoV, alors que le MERS-CoV était plutôt inhibé par certains inhibiteurs de transport d'ions, inhibiteurs de la tubuline, ou inhibiteurs d'apoptose. Sur 290 composés testés, seuls 33 composés présentant une activité antivirale sur le MERS-CoV ont été identifiés en culture cellulaire.

Toutefois, de nombreuses données de la littérature indiquent que les composés antiviraux efficaces sur le SARS-CoV ne sont pas systématiquent actifs contre le MERS-CoV, et inversement ; ces virus présentant des différences en termes à la fois de composition protéique et d'interactions fonctionnelles avec la cellule hôte. (Dijkman & van der Hoek 2009 ; Dyall *et al.,* 2014)

Une stratégie alternative pour identifier de nouvelles molécules thérapeutiques est de tester des molécules ayant une action sur les voies de signalisation, biogénèse et métaboliques cellulaires que ciblent et/ou détournent à leur profit les virus pour mener à bien leur cycle réplicatif. Cette stratégie, présentée dans l'article de Josset et al. (Plos One, 2010), a déjà permis l'identification de molécules présentant une activité antivirale contre les virus influenza, telles que les molécules présentées dans les demandes de brevet FR 2 953 410, FR 3 033 701 et FR 3 049 861.

Par ailleurs, Josset et collaborateurs ont mis en évidence le fait que le MERS-CoV induit un profil d'expression génique des cellules cibles qu'il infecte, tout à fait différent de celui induit par le SARS-CoV. Selon cette étude, les inhibiteurs de glucocorticoïdes et de kinases pourraient être des classes pharmaceutiques à privilégier pour la recherche de composés antiviraux actifs sur le coronavirus MERS-COV. (Josset *et al.,* 2013).

A l'heure actuelle, il n'existe pas, ou très peu, de molécules thérapeutiques reconnues et/ou approuvées par les autorités de santé pour lutter contre les infections par le virus MERS-CoV.

Par ailleurs, il n'existe sur le marché aucun vaccin contre le virus MERS-CoV. Quelques candidats sont actuellement évalués en essai clinique de phase I (Phase I, Open Label Dose Ranging Safety Study of GLS-5300 in Healthy Volunteers - Full Text View - ClinicalTrials.gov," accessed March 24, 2016, https://clinicaltrials.gov/ct2/show/NCT02670187?term=MERS+coronavirus&rank=7).

Dans ces conditions, il existe un besoin pour de nouvelles solutions prophylactiques et/ou thérapeutiques à l'encontre d'infections par le coronavirus MERS-COV.

### RESUME DE L'INVENTION

Dans le cadre de la présente invention, plusieurs composés ont été sélectionnés et évalués dans un test cellulaire d'infection virale. Certains composés ont ainsi présenté un effet antiviral inattendu sur le coronavirus MERS-CoV.

Les composés sélectionnés avaient été décrits préalablement pour d'autres applications thérapeutiques. De façon surprenante, il a maintenant été montré que ces composés, seuls ou en combinaison, ont une activité antivirale contre le MERS-CoV, et permettent le traitement et/ou la prévention des infections par ce coronavirus.

L'invention concerne des compositions pharmaceutiques ou vétérinaires pour leur utilisation dans la prévention et/ou le traitement d'une infection par le coronavirus MERS-COV (Middle-East Respiratory Syndrome), caractérisée en ce qu'elles comprennent, dans un véhicule pharmaceutique approprié, au moins un composé choisi parmi l'Apigénine et la Berbérine.

L'invention est également relative à une composition pharmaceutique ou vétérinaire pour son utilisation dans la prévention et/ou le traitement d'une infection par le coronavirus MERS-COV (Middle-East Respiratory Syndrome), caractérisée en ce qu'elle comprend, dans un véhicule pharmaceutique approprié, au moins un composé choisi parmi l'apigénine, la berbérine et la monensine, ou une combinaison d'au moins deux de ces composés.

Ces compositions pharmaceutiques ou vétérinaires peuvent avantageusement comprendre en outre au moins un autre agent antiviral, et/ou un agent antibacterien. Au sens de l'invention, un autre agent antiviral est un agent distinct de l'apigénine, de la berbérine et de la monensine. Egalement, au sens de l'invention, un agent antibactérien est un agent distinct de l'apigénine, de la berbérine et de la monensine.

L'invention a également pour objet des compositions pharmaceutiques ou vétérinaires comprenant au moins un agent antiviral en combinaison avec au moins un composé choisi parmi l'apigénine, la berbérine et la monensine, ou une combinaison de deux de ces composés, ou une combinaison de ces trois composés.

En particulier, ladite composition comprend dans un véhicule pharmaceutique approprié, au moins deux composés choisis parmi :
(a) un agent antiviral ; et
(b) un composé choisi parmi l'apigénine, la berbérine, et une combinaison des deux.

L'invention a également pour objet des compositions pharmaceutiques ou vétérinaires, comprenant dans un véhicule pharmaceutique approprié, une combinaison de berbérine et de monensine.

Selon un aspect particulier de l'invention, lesdites compositions sont sous une forme galénique destinée à une administration par inhalation

### FIGURES

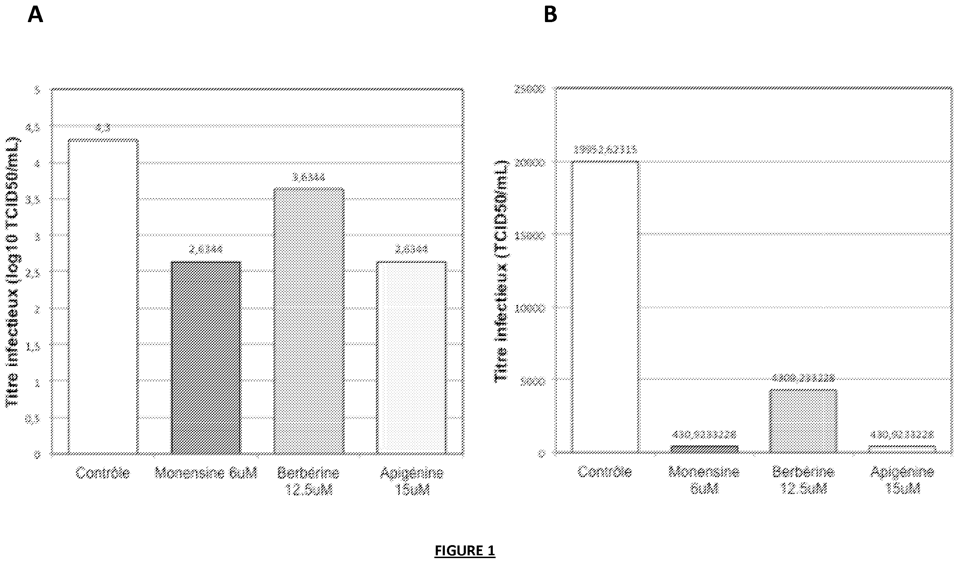

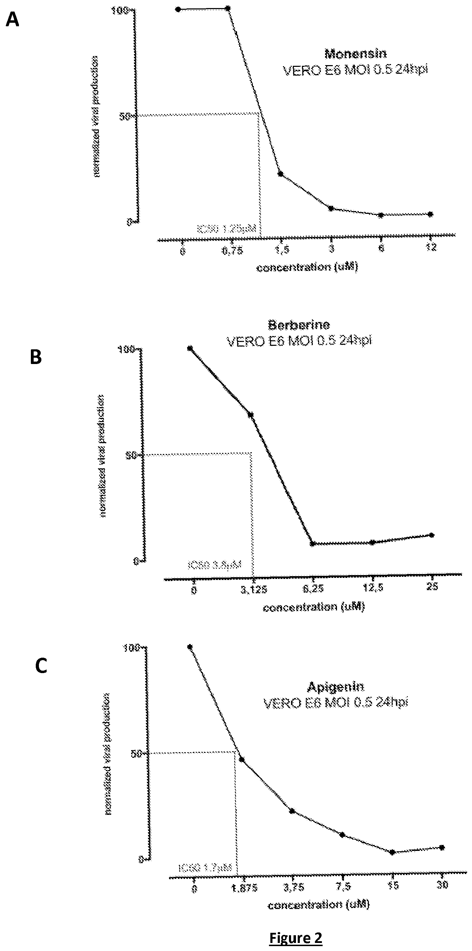

**Figure 1****.** Mesure des titres infectieux de MERS-CoV à 24 heures post-infection, sur des cellules traitées (par monensine, berbérine ou apigénine), rapportés au titre infectieux des cellules témoins infectées et non traitées. Les titres infectieux ont été obtenus par technique d'infection par dilution limite et calcul par la méthode de Reed et Muench.
   **A)** Les résultats sont exprimés en log 10 TCID/mL (50% Tissue Culture Infective Dose)
   **B)** Les résultats sont exprimés en TCID/mL
**Figure 2****.** Mesure des IC50 (concentration nécessaire pour obtenir 50% de production virale normalisée) des composés testés (**A-** monensine, **B-** berbérine, **C-** apigénine) sur des cellules VeroE6 infectées par le coronavirus MERS-COV. Les titres infectieux ont été obtenus par technique d'infection par dilution limite et calcul par la méthode de Reed et Muench.

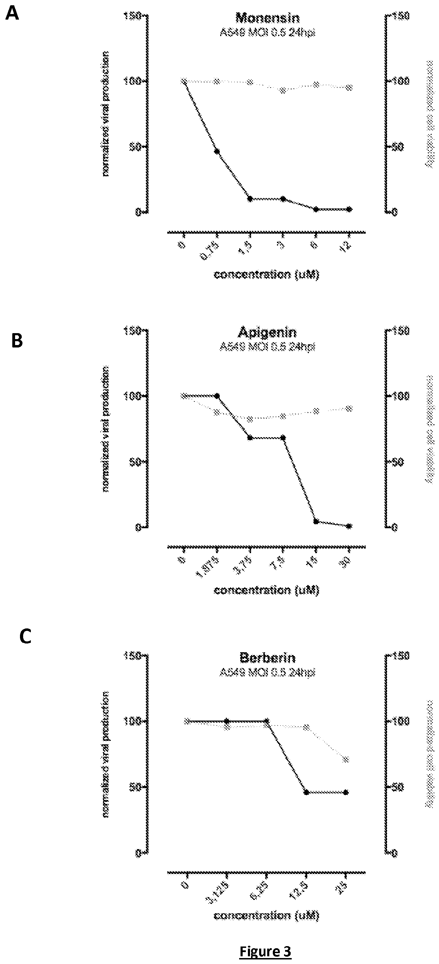

**Figure 3****.** Mesure d'efficacité antivirale des composés testés (**A-** monensine, **B-** apigénine, **C**-berbérine) sur des cellules humaines épithéliales respiratoires A549 infectées par le coronavirus MERS-COV : Les titres infectieux ont été obtenus par technique d'infection par dilution limite et calcul par la méthode de Reed et Muench. La production virale normalisée est mesurée en parallèle de la viabilité des cellules, en fonction de la concentration de composés utilisés.

### DESCRIPTION DETAILLEE DE L'INVENTION

La présente invention concerne une composition pharmaceutique ou vétérinaire pour son utilisation dans la prévention et/ou le traitement d'une infection par le coronavirus MERS-CoV (Middle-East Respiratory Syndrome), caractérisée en ce qu'elle comprend, dans un véhicule pharmaceutique approprié, au moins un composé choisi parmi l'Apigénine et la Berbérine.

Les compositions selon l'invention sont en particulier destinées à une utilisation dans le traitement d'une infection par le coronavirus MERS-COV.

Par « infection par le coronavirus MERS-CoV » on désigne le fait qu'un organisme, humain ou animal, présente des cellules ayant été infectées par le coronavirus MERS-CoV. L'infection peut notamment être établie en effectuant une détection et/ou une titration virale à partir de prélèvements respiratoires, ou en dosant les anticorps spécifiques du MERS-CoV circulants dans le sang. La détection chez les individus infectés de ce virus spécifique est réalisée par des méthodes de diagnostic classiques, notamment de biologie moléculaire (PCR), bien connues de l'Homme du métier.

Le terme « traitement » désigne le fait de combattre l'infection par le coronavirus MERS-CoV dans un organisme humain ou animal. Grâce à l'administration d'au moins une composition selon l'invention, le taux d'infection virale (titre infectieux) dans l'organisme va diminuer, et de préférence le virus va disparaitre complètement de l'organisme. Le terme « traitement » désigne aussi le fait d'atténuer les symptômes associés à l'infection virale (syndrome respiratoire, défaillance rénale, fièvre, etc...).

Les compositions selon l'invention sont également destinées à une utilisation dans la prévention d'une infection par le MERS-CoV.

Au sens de l'invention, le terme « prévention » désigne le fait d'empêcher, ou du moins de diminuer la probabilité d'apparition, d'une infection dans un organisme humain ou animal par le MERS-CoV. Grâce à l'administration d'au moins une composition selon l'invention, les cellules humaines ou animales dudit organisme deviennent moins permissives à l'infection, et sont ainsi plus à même de ne pas être infectées par ledit coronavirus.

Les compositions selon l'invention peuvent être de type pharmaceutique, destinées à être administrées à un être humain, ou de type vétérinaire, destinées à être administrés à des animaux non humains.

Concernant les animaux, il est entendu que les compositions vétérinaires pour leur utilisation dans la prévention et/ou le traitement d'une infection par le coronavirus MERS-CoV (Middle-East Respiratory Syndrome) sont destinées à être administrées à des animaux infectés par ce coronavirus. Ces compositions sont notamment destinées aux mammifères, notamment aux animaux de rente, et en particulier aux mammifères de la famille des camélidés et tout particulièrement au chameau (genre *Camelus*).

Selon l'invention, le terme « véhicule pharmaceutique approprié » désigne des véhicules ou des excipients, c'est à dire des composés ne présentant pas d'action propre sur l'infection ici considérée. Ces véhicules ou excipients sont pharmaceutiquement acceptables, ce qui signifie qu'ils peuvent être administrés à un individu ou à un animal sans génération d'effets délétères significatifs.

Selon un premier aspect, la composition pharmaceutique ou vétérinaire pour son usage selon l'invention comprend au moins une quantité efficace d'apigénine.

Selon un second aspect, la composition pharmaceutique ou vétérinaire pour son usage selon l'invention comprend au moins une quantité efficace de berbérine.

Par « quantité efficace », on entend au sens de l'invention une quantité de composé actif suffisante pour inhiber la prolifération et/ou la réplication du coronavirus, et/ou le développement de l'infection virale au sein de l'organisme. Cette inhibition peut être quantifiée, par exemple en mesurant le titre viral comme cela est présenté dans les exemples de la présente demande.

Selon un aspect préféré de l'invention, ladite composition pharmaceutique ou vétérinaire pour son utilisation telle que citée ci-dessus est caractérisée en ce qu'elle comprend une combinaison d'au moins deux des composés suivants : apigénine, berbérine et monensine.

Ladite composition pharmaceutique pourra notamment comprendre l'une des combinaisons suivantes :
- Apigénine et Berbérine ;
- Apigénine et Monensine ;
- Berbérine et Monensine ; ou
- Apigénine, Berbérine et Monensine.

Selon un aspect particulier de l'invention, la composition pharmaceutique pour son utilisation selon l'invention consistera en l'une des combinaisons de composés telles que citées ci-dessus, et un véhicule pharmaceutique adapté.

Ces composés sont connus pour une utilisation dans d'autres applications thérapeutiques sans rapport direct avec une activité antivirale contre le coronavirus MERS-CoV, comme cela est présenté ci-dessous.

### L'apigénine

L'apigénine est un composé naturel de la famille des flavones (flavonoïdes), que l'on trouve dans le persil, le plantain, l'achillée millefeuille, la passiflore ou encore le romarin.

Sa structure chimique développée est schématisée ci-dessous :

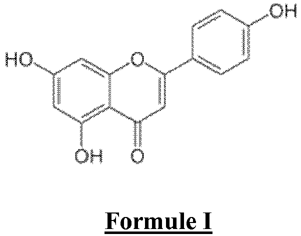

Cette molécule est connue pour ses propriétés anti-inflammatoires, même si son mode d'action n'est pas connu en détail. Certains travaux indiquent que l'apigénine agit en inhibant le cytochrome P450.

L'apigénine a montré son efficacité contre certains virus, comme le virus HVC de l'hépatite C (Shibata *et al.* 2014), l'entérovirus 71 (Lv *et al.* 2014) ou encore le virus gamma de l'herpès (WO 2016/013793).

### La berbérine

La berbérine est un alcaloïde naturel que l'on retrouve dans un grand nombre de végétaux.

Sa structure chimique développée est schématisée ci-dessous :

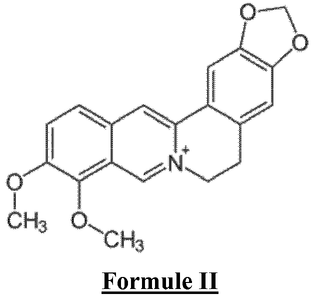

Cette molécule est largement utilisée dans la pharmacopée asiatique, pour ses propriétés antifongiques, antibactériennes et anti-inflammatoires.

Son mécanisme d'action, bien que non complètement élucidé, relèverait essentiellement de l'activation de l'AMP Kinase (Adenosine Monophosphate activated Protein Kinase). Cette enzyme ubiquitaire joue un rôle dans l'homéostasie énergétique cellulaire. L'activation de l'AMPK a principalement pour effet de (i) stimuler l'oxydation des acides gras hépatiques et la cétogenèse, (ii) inhiber la synthèse du cholestérol, la lipogénèse et la synthèse des triglycérides, (iii) stimuler l'oxydation des acides gras dans les muscles squelettiques et l'absorption du glucose par les muscles et (iv) moduler la sécrétion d'insuline par les cellules bêta du pancréas.

La berbérine présente une activité antivirale sur le virus HSV (Song et al. 2014) et sur les virus influenza (Wu et al. 2011).

De plus, la berbérine pourrait présenter une action antivirale contre le SARS-CoV (http://apps.who.int/medicinedocs/fr/d/Js6170e/ et WO 2013/185126), bien qu'aucune donnée expérimentale ne soit disponible.

### La monensine

La monensine est un antibiotique polyéther isolé chez une bactérie du genre Streptomyces. Cette molécule est très largement utilisée comme additif dans l'alimentation des ruminants (sous le nom commercial Rumensin®), pour prévenir des infections de type coccidiosc.

Sa structure chimique développée est schématisée ci-dessous :

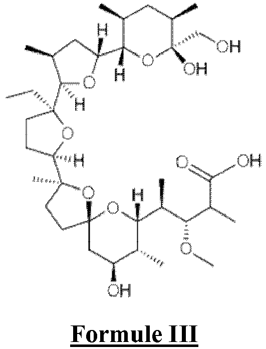

La monensine agirait comme un ionophore, en perturbant le trafic intracellulaire.

Cette molécule est connue pour présenter une cytotoxicité importante lorsqu'elle est utilisée à de hautes concentrations. Chez l'animal, des différences très importantes de DL50 (Dose Létale 50%) existent. Par exemple, chez les chevaux, la DL50 est 100 fois moindre que chez les ruminants, ce qui pose de très gros problèmes d'intoxication par contamination croisée de la nourriture dans certaines fermes.

Un nombre croissant de données de la littérature montre également une activité anti-paludéenne, ainsi qu'une activité antivirale contre plusieurs virus : contre les virus influenza (FR 3 033 701 et FR 3 049 861) et plus particulièrement contre les virus enveloppés, dont certains virus membre de la famille des coronavirus.

La demande de brevet US 2009/0149545 cite de nombreux composés, et notamment la monensine, pour son action thérapeutique contre l'infection par coronavirus.

La demande de brevet WO 2015/157223, relative à la recherche de composés ayant une activité antivirale contre les coronavirus, rapporte que la monensine pourrait être utilisée contre le MERS-CoV, sans toutefois présenter de résultats expérimentaux permettant d'établir cette conclusion.

### Agent antiviral

Au sens de la présente invention, on entend par les termes « agent antiviral » ou « composé antiviral » des agents actifs qui agissent sur la charge virale (aussi dénommé titre infectieux), en inhibant soit de manière directe soit de manière indirecte la réplication et/ou la dissémination du coronavirus MERS-CoV, au sein d'un organisme infecté.

Par « activité antivirale » ou « action antivirale », on entend une action sur le virus ou sur ses cellules cibles, notamment l'action d'inhiber le cycle de réplication du virus ou sa capacité à infecter et à se reproduire dans des cellules hôtes, cet effet antiviral pouvant être obtenu par la modulation d'un certain nombre de gènes des cellules cibles.

On entend par 'cellules cibles' des cellules infectées par le coronavirus et/ou susceptibles d'être infectées prochainement, de par leur proximité immédiate avec des cellules infectées.

### Combinaison avec un autre agent actif

Il est entendu que la composition pour son utilisation selon l'invention comprend de l'apigénine et/ou de la berbérine, et qu'elle peut donc également comprendre d'autres composés actifs, en sus du véhicule pharmaceutique approprié.

En effet, l'apigénine, la berbérine et la monensine, ou leurs mélanges, peuvent être employés en thérapie seuls, ou en combinaison avec au moins un autre agent actif.

Il peut s'agir de composés permettant d'améliorer l'activité de ces composés, ou encore d'autres actifs connus pour une activité particulière.

Ces composés actifs supplémentaires pourront être choisis parmi les classes pharmaceutiques d'agents cités dans la demande WO 2015/157223, à savoir parmi les agents antibactériens, les agents antiparasitaires, les inhibiteurs de la neurotransmission, les inhibiteurs du récepteur aux oestrogènes, les inhibiteurs de synthèse et réplication de l'ADN, les inhibiteurs de la maturation des protéines, les inhibiteurs des voies kinase, les inhibiteurs du cytosquelette, les inhibiteurs du métabolisme des lipides, les agents anti-inflammatoires, les inhibiteurs des canaux ioniques, les inhibiteurs d'apoptose et les inhibiteurs de cathepsine.

Ces composés actifs pourront notamment être choisis parmi des agents antibactériens, des inhibiteurs de canaux ioniques, des agents immunosuppresseurs et des agents antiviraux. On peut par exemple citer comme agent antiviral l'acyclovir.

Selon un aspect particulier de l'invention, la composition pharmaceutique ou vétérinaire pour son utilisation dans la prévention et/ou le traitement d'une infection par le coronavirus MERS-CoV, comprend, outre de l'apigénine et/ou de la berbérine, au moins un autre agent antiviral.

Selon un autre aspect particulier de l'invention, la composition pharmaceutique ou vétérinaire pour son utilisation dans la prévention et/ou le traitement d'une infection par le coronavirus MERS-CoV, comprend, outre une combinaison d'au moins deux composés choisis parmi l'apigénine, la berbérine et la monensine, au moins un autre agent antiviral.

Il est entendu que cet agent antiviral sera utilisé aux doses nécessaires pour présenter une action antivirale, cette dose étant désignée comme étant « efficace », ce dosage pouvant être facilement déterminé par l'homme du métier.

Au sens de l'invention, un agent antiviral désigne un composé agissant sur un virus, en inhibant et/ou ralentissant et/ou prévenant l'infection virale associée.

Les agents antiviraux sont classifiés en différentes catégories en fonction de leur mode d'action. On peut citer notamment :
- les analogues de nucléotides, qui interfèrent ou stoppent la synthèse d'ADN ou d'ARN ; ainsi que les inhibiteurs des enzymes impliquées dans la synthèse d'ADN ou d'ARN (hélicase, réplicase) ;
- les composés qui inhibent les étapes de maturation du virus au cours de son cycle de réplication ;
- les composés qui interfèrent avec la liaison à la membrane cellulaire, ou à l'entrée des virus dans des cellules hôtes (Inhibiteurs de fusion ou d'entrée) ;
- les agents qui empêchent le virus de s'exprimer au sein de la cellule hôte après son entrée, en bloquant son désassemblage au sein de la cellule ;
- les agents qui restreignent la propagation des virus vers d'autres cellules.

On peut citer en particulier les agents antiviraux bien connus de l'Homme du métier, destinés à lutter contre des virus à ARN, tels que les inhibiteurs de protéase, les inhibiteurs d'hélicase, et les inhibiteurs d'entrée cellulaire du virus MERS-CoV dans les cellules cibles.

Parmi les agents antiviraux bien connus de l'Homme du métier, on peut citer de manière plus précise la ribavirine, un analogue nucléosidique de la guanosine à large spectre antiviral ; ainsi que l'interféron qui agit en inhibant la réplication virale dans les cellules de l'hôte.

Selon un aspect particulier de l'invention, la composition pharmaceutique ou vétérinaire est caractérisée en ce que l'autre agent antiviral est choisi parmi les composés suivants : la ribavirine, un interféron, ou une combinaison des deux.

Au sens de l'invention, on entend par « un interféron » ou « l'interféron » un composé appartenant à la famille des interférons, qui sont des glycoprotéines sécrétées par les cellules du système immunitaire.

Les interférons sont une famille de petites molécules protéiques d'un poids moléculaire d'environ 15 000 à 21 000 daltons. Trois classes majeures d'interférons ont été identifiées : alpha, bêta et gamma. Ces 3 classes principales ne sont pas elles-mêmes homogènes et peuvent regrouper plusieurs espèces moléculaires différentes d'interféron. Plus de 14 interférons alfa humains génétiquement différents ont été identifiés.

Il est entendu que dans la composition pharmaceutique ou vétérinaire selon l'invention, l'interféron utilisé sera un polypeptide recombinant, synthétisé en laboratoire.

En particulier, l'interféron utilisé sera l'interféron alfa-2b recombinant dont l'efficacité sur la réplication virale *in vitro* et *in vivo* a été démontrée.

De tels agents antiviraux sont disponibles dans le commerce, et leurs conditions d'utilisation sont décrites dans des ouvrages de référence comme Le Dictionnaire Vidal.

Ainsi, la composition pharmaceutique pour son utilisation selon l'invention pourra comprendre l'une des combinaisons suivantes :
- Apigénine, berbérine et ribavirine ;
- Apigénine, berbérine et interféron ;
- Apigénine, berbérine, interféron et ribavirine ;
- Apigénine, monensine et ribavirine ;
- Apigénine, monensine et interféron ;
- Apigénine, monensine, interféron et ribavirine ;
- Berbérine, monensine et ribavirine ;
- Berbérine, monensine et interféron ;
- Berbérine, monensine, interféron et ribavirine ;
- Apigénine, berbérine, monensine et ribavirine ;
- Apigénine, berbérine, monensine et interféron ;
- Apigénine, berbérine, monensine, interféron et ribavirine.

Selon un aspect particulier de l'invention, la composition pharmaceutique pour son utilisation selon l'invention consistera en une combinaison d'agents actifs telles que citées ci-dessus, et un véhicule pharmaceutique adapté.

### Mode d'administration des compositions pharmaceutiques ou vétérinaires

Les compositions pharmaceutiques et vétérinaires pour l'utilisation selon la présente invention sont adaptées pour une administration orale, sublinguale, par inhalation, sous-cutanée, intramusculaire, intraveineuse, transdermique, oculaire ou rectale.

Selon une mise en œuvre particulière de l'invention, la composition pharmaceutique ou vétérinaire pour son utilisation selon l'invention est caractérisée en ce qu'elle est sous une forme galénique destinée à une administration par inhalation.

L'inhalation désigne l'absorption par les voies respiratoires. C'est en particulier une méthode d'absorption de composés à des fins thérapeutiques, de certaines substances sous forme de gaz, de micro-gouttelettes ou de poudre en suspension.

L'administration de compositions pharmaceutiques ou vétérinaires par inhalation, c'est-à-dire par les voies nasale et/ou buccale, est bien connue de l'Homme du métier.

On distingue deux types d'administration par inhalation :
- l'administration par insufflation lorsque les compositions sont sous la forme de poudres, et
- l'administration par nébulisation lorsque les compositions sont sous la forme d'aérosols (suspensions) ou sous la forme de solutions, par exemple de solutions aqueuses, mises sous pression. L'utilisation d'un nébuliseur ou d'un pulvérisateur sera alors recommandée pour administrer la composition pharmaceutique ou vétérinaire.

La forme galénique considérée ici est donc choisie parmi : une poudre, une suspension aqueuse de gouttelettes ou une solution sous pression.

### Produit de combinaison

La présente invention concerne également un produit de combinaison comprenant :
- une composition selon l'invention, comprenant au moins un composé choisi parmi l'apigénine et la berbérine, optionnellement en présence de monensine, et
- au moins un agent antiviral,
pour une utilisation simultanée, séparée ou séquentielle pour prévenir et/ou traiter une infection par le coronavirus MERS-CoV, chez l'homme ou chez l'animal.

Un tel produit de combinaison pourra être utilisé dans la prévention et/ou le traitement d'une infection par le coronavirus MERS-CoV, dans le cadre d'une utilisation simultanée, séparée ou séquentielle. Ainsi, les au moins deux agents actifs compris dans le produit de combinaison pourront être administrés de manière simultanée, séparée ou séquentielle.

Il est entendu que toutes les combinaisons de deux, trois ou quatre composés actifs citées précédemment peuvent chacune être sous la forme d'un produit de combinaison, c'est-à-dire que les deux, trois ou quatre composés actifs peuvent être administrés de manière simultanée, séparée ou séquentielle, pour prévenir et/ou traiter une infection par le coronavirus MERS-CoV.

L'invention se rapporte également à une méthode thérapeutique pour la prévention et/ou le traitement d'une infection par le coronavirus MERS-CoV chez l'homme, dans laquelle on administre à un patient une quantité efficace d'un composé choisi parmi l'apigénine et la berbérine, ou un mélange des deux, optionnellement en association avec de la monensine.

L'invention se rapporte également à une méthode thérapeutique pour la prévention et/ou le traitement d'une infection par le coronavirus MERS-CoV chez les animaux dans laquelle on administre à un animal une quantité efficace d'un composé choisi parmi l'apigénine et la berbérine, optionnellement en association avec de la monensine.

L'invention se rapporte également à l'utilisation d'un composé choisi parmi l'apigénine et la berbérine, optionnellement en association avec de la monensine, pour la préparation d'une composition pharmaceutique destinée à la prévention et/ou le traitement d'une infection par le coronavirus MERS-CoV.

### Compositions pharmaceutiques ou vétérinaires

La présente invention concerne également une composition pharmaceutique ou vétérinaire, comprenant dans un véhicule pharmaceutique approprié, au moins deux composés choisis parmi :
(a) un agent antiviral ; et
(b) un composé choisi parmi l'apigénine, la berbérine, et une combinaison des deux.

En particulier, l'agent antiviral est destiné à lutter contre une infection par le coronavirus MRES.

L'agent viral pourra notamment être choisi parmi :
- les analogues de nucléotides, qui interfèrent ou stoppent la synthèse d'ADN ou d'ARN ; ainsi que les inhibiteurs des enzymes impliquées dans la synthèse d'ADN ou d'ARN (hélicase, réplicase) ;
- les composés qui inhibent les étapes de maturation du virus au cours de son cycle de réplication ;
- les composés qui interfèrent avec la liaison à la membrane cellulaire, ou à l'entrée des virus dans des cellules hôtes (Inhibiteurs de fusion ou d'entrée) ;
- les agents qui empêchent le virus de s'exprimer dans la cellule hôte après son entrée, en bloquant son désassemblage au sein de la cellule ;
- les agents qui restreignent la propagation des virus vers d'autres cellules.

On peut citer en particulier les agents antiviraux bien connus de l'Homme du métier, destinés à lutter contre des virus à ARN, tels que les inhibiteurs de protéase, les inhibiteurs d'hélicase, et les inhibiteurs d'entrée du virus dans les cellules cibles.

Parmi les agents antiviraux bien connus de l'Homme du métier, on peut citer de manière plus précise la ribavirine, un analogue nucléosidique de la guanosine à large spectre antiviral ; ainsi que l'interféron qui agit en inhibant la réplication virale dans les cellules de l'hôte.

Selon un aspect particulier de l'invention, la composition contient, en tant qu'agent antiviral, un interféron, de la ribavirine, ou une combinaison des deux.

La composition pharmaceutique selon l'invention pourra également comprendre de la monensine.

Ainsi, la composition pharmaceutique selon l'invention pourra comprendre l'une des combinaisons suivantes :
- Apigénine et ribavirine ;
- Apigénine et interféron ;
- Apigénine, interféron et ribavirine ;
- Berbérine et ribavirine ;
- Berbérine et interféron ;
- Berbérine, interféron et ribavirine ;
- Apigénine, berbérine et ribavirine ;
- Apigénine, berbérine et interféron ;
- Apigénine, berbérine, interféron et ribavirine ;
- Apigénine, monensine et ribavirine ;
- Apigénine, monensine et interféron ;
- Apigénine, monensine, interféron et ribavirine ;
- Berbérine, monensine et ribavirine ;
- Berbérine, monensine et interféron ;
- Berbérine, monensine, interféron et ribavirine ;
- Apigénine, berbérine, monensine et ribavirine ;
- Apigénine, berbérine, monensine et interféron ;
- Apigénine, berbérine, monensine, interféron et ribavirine.

Selon un aspect particulier de l'invention, la composition pharmaceutique ou vétérinaire selon l'invention consistera en une combinaison d'agents actifs telles que citées ci-dessus, et un véhicule pharmaceutique adapté.

Plus particulièrement, la composition pharmaceutique ou vétérinaire selon l'invention comprendra, dans un véhicule pharmaceutique approprié, au moins une combinaison d'un interféron, de monensine, d'apigénine et de berbérine.

De telles compositions pourront être utilisées dans toute sorte d'utilisation thérapeutique ou prophylactique, et seront en particulier utilisées dans le traitement et/ou la prévention des infections virales.

Les compositions selon l'invention peuvent être de type pharmaceutique, destinées à être administrées à un être humain, ou de type vétérinaire, destinées à être administrés à des animaux non humains.

Concernant les animaux, les compositions vétérinaires selon l'invention sont notamment destinées aux mammifères, notamment aux animaux de rente, et en particulier aux mammifères de la famille des camélidés, et tout particulièrement au chameau (genre *Camelus*).

Il est à noter toutefois le fait que la monensine présente une forte toxicité chez le cheval (genre *Equus*) et que les compositions vétérinaires selon l'invention comprenant de la monensine ne sont pas destinées à une administration aux mammifères appartenant au genre *Equus.*

De telles compositions pourront notamment être utilisées dans le traitement et/ou la prévention des infections par des coronavirus, et tout particulièrement par le coronavirus MERS-CoV.

Selon une mise en œuvre particulière de l'invention, de telles compositions pourront être sous une forme galénique destinée à une administration par inhalation.

### Combinaison berbérine et monensine

La présente invention concerne également une composition pharmaceutique ou vétérinaire, comprenant dans un véhicule pharmaceutique approprié, une combinaison de berbérine et de monensine.

Ladite composition pourra être utilisée dans toute sorte d'utilisation thérapeutique ou prophylactique, et sera en particulier utilisée dans le traitement et/ou la prévention des infections virales.

Une telle composition comprenant une combinaison de berbérine et de monensine pourra notamment être utilisée dans le traitement et/ou la prévention des infections par des coronavirus, et tout particulièrement par le coronavirus MERS-CoV.

Selon une mise en œuvre particulière de l'invention, cette composition pourra être sous une forme galénique destinée à une administration par inhalation.

### EXEMPLES

Les exemples présentés ci-dessous démontrent que les composés cités précédemment présentent, de façon inattendue, une activité antivirale significative contre MERS-CoV, *in vitro* sur deux modèles distincts de cellules de mammifères, dans des conditions de cytotoxicité modérées.

Dans un premier temps, les composés ont été testés *in vitro,* à une concentration classiquement utilisée dans la littérature, dans le contexte d'une infection de cellules VERO-E6 par la souche de MERS-CoV France-UAE/1612/2013, N° accession Genbank KF745068.

Les cellules VERO-E6 sont issues de cellules épithéliales de rein de singe, et sont sensibles à l'infection par le MERS-CoV. Elles constituent un des modèles cellulaires d'études largement utilisé dans la littérature.

Les paramètres d'infection ont été déterminés au préalable afin d'obtenir les meilleures conditions pour observer un possible effet antiviral.

Les cellules VERO-E6 ont été infectées à une MOI (Multiplicity Of Infection) de 0.5 pour une durée de 24h à 37 °C sous 5 % de CO2, en présence ou absence de composés testés.

Après 24h d'incubation, les titres infectieux (log10 DITC50/ml ou DITC50/mL) ont été déterminés dans les surnageants de culture de cellules infectées.

Les titres infectieux sont indiqués TCID50/mL (50% Tissue Culture Infective Dose) en Fig1A (log10 TCID50/mL) et Fig 1B (TCID50/mL). Ils ont été obtenus par technique d'infection par dilution limite et calcul par la méthode de Reed et Muench.

Un test de cytotoxicité et un test de quantification du coronavirus sont réalisés après 24 h d'incubation. La cytotoxicité des différents composés est déterminée à chaque essai dans une plaque de cellules non infectées par un test de viabilité (Test MTS, Promega). Ce test est basé sur la mesure de l'activité métabolique des cellules, qui transforme un substrat (MTS tetrazolium) en un produit (Formazan), soluble dans le milieu et dont l'absorbance mesurée à 490 nm reflète proportionnellement le nombre de cellules vivantes. Le rapport de l'absorbance dans chaque puits sur l'absorbance moyenne des puits des cellules témoins (non traitées par les composés) est calculé et indiqué sur les schémas comme un indice de viabilité cellulaire (viabilité cellulaire relative).

L'effet sur la production virale *in vitro* est mesuré par détermination des titres infectieux (TCID50/mL) réalisée sur des cellules VERO-E6, les titres étant calculés selon la technique de Reed et Muench. Le rapport du titre infectieux dans chaque condition a été exprimé en fonction du titre infectieux mesuré dans la condition contrôle (sans traitement).

Les résultats sont présentés en figures 1A (titres infectieux exprimés en log10 TCID50/mL) et 1B (titres exprimés en TCID50/mL).

Les titres infectieux mesurés dans les conditions expérimentales en présence des composés testés sont fortement réduits par rapport à la condition contrôle, où les cellules ont été infectées mais non traitées.

En effet, la monensine (6 µM), la berbérine (12,5 µM) et l'apigénine (15 µM) permettent une réduction de 97,8%, 78,4% et 97,8% respectivement des titres infectieux par rapport au contrôle (cf. Figure 1).

A partir de ces résultats, une gamme plus large de concentrations a été testée pour chacun des trois composés sur des cellules VeroE6 dans les mêmes conditions expérimentales, afin de déterminer la gamme de concentration efficace, et ainsi déterminer une IC50 (half maximal Inhibitory Concentration) c'est-à-dire la dose nécessaire pour obtenir 50% d'inhibition de la production virale (cf. Figure 2).

Il a été possible de déterminer une valeur d'IC50 pour ces trois composés :
- 1.25 µM, pour la monensine
- 3,8 µM pour la berbérine et
- 1,7 µM pour l'apigénine.

Ces valeurs d'IC50 sont relativement faibles par rapport aux concentrations usuelles connues pour ces composés dans leurs applications hors champs infectieux. Ces valeurs sont également très éloignées des concentrations de cytotoxicité (CC50).

Des résultats similaires d'efficacité ont également été obtenues pour ces trois composés dans un autre modèle cellulaire de choix, celui de la lignée cellulaire épithéliale respiratoire humaine A549 (figure 3).

### REFERENCES BIBLIOGRAPHIQUES

### BREVETS

FR 2 953 410
FR 3 033 701
FR 3 049 861
WO 2016/013793
WO 2013/185126
US 2009/0149545
WO 2015/157223

### NON BREVETS

Emmie de Wit, Neeltje van Doremalen, Darryl Falzarano & Vincent J. Munster. SARS and MERS: recent insights into emerging coronaviruses. Nature Reviews Microbiology. 14, 523-534 (2016).
R. Dijkman, L. van der Hoek. Human coronaviruses 229E and NL63: close yet still so far. J Formos Med Assoc 108 (2009), pp. 270-279
Dyall J, Coleman CM, Hart BJ, Venkataraman T, Holbrook MR, Kindrachuk J, Johnson RF, Olinger GG Jr, Jahrling PB, Laidlaw M, Johansen LM, Lear-Rooney CM, Glass PJ, Hensley LE, Frieman MB. Repurposing of clinically developed drugs for treatment of Middle East respiratory syndrome coronavirus infection. Antimicrob Agents Chemother. 2014 Aug;58(8):4885-93.
Jaffar A. Al-Tawfiq et al., Ribavirin and Interferon Therapy in Patients Infected with the Middle East Respiratory Syndrome Coronavirus: An Observational Study. International Journal of Infectious Diseases: IJID: Official Publication of the International Society for Infectious Diseases 20 (March 2014): 42-46
Josset L, Menachery VD, Gralinski LE, Agnihothram S, Sova P, Carter VS, Yount BL, Graham RL, Baric RS, Katze MG. Cell host response to infection with novel human coronavirus EMC predicts potential antivirals and important différences with SARS coronavirus.MBio. 2013 Apr 30;4
Adedeji AO, Sarafianos SG. Antiviral drugs specific for coronaviruses in preclinical development.Curr Opin Virol. 2014 Oct;8:45-53.
Kilianski A, Baker SC. Cell-based antiviral screening against coronaviruses: developing virus-specific and broad-spectrum inhibitors. Antiviral Res. 2014 Jan; 101:105-12.
Shibata C, Ohno M, Otsuka M, Kishikawa T, Goto K, Muroyama R, Kato N, Yoshikawa T, Takata A, Koike K. The flavonoid apigenin inhibits hepatitis C virus replication by decreasing mature microRNA122 levels. Virology. 2014 Aug;462-463:42-8.
Lv X, Qiu M, Chen D, Zheng N, Jin Y, Wu Z. Apigenin inhibits enterovirus 71 replication through suppressing viral IRES activity and modulating cellular JNK pathway. Antiviral Res. 2014 Jun 24;109C:30-41.)
Song S, Qiu M, Chu Y, Chen D, Wang X, Su A, Wu Z. Berberine down-regulates cellular JNK and NF-κB activation and this may result in an inhibition of HSV replication. Antimicrob Agents Chemother. 2014 Jun 9.
Wu Y, Li JQ, Kim YJ, Wu J, Wang Q, Hao Y. In vivo and in vitro antiviral effects of berberine on influenza virus. Chin J Integr Med. 2011 Jun;17(6):444-52.

## Revendications

1. Composition pharmaceutique ou vétérinaire pour son utilisation dans la prévention et/ou le traitement d'une infection par le coronavirus MERS-CoV (Middle-East Respiratory Syndrome), **caractérisée en ce qu'**elle comprend, dans un véhicule pharmaceutique approprié, au moins un composé choisi parmi l'Apigénine et la Berbérine.

2. Composition pharmaceutique ou vétérinaire pour son utilisation selon la revendication 1, **caractérisée en ce qu'**elle comprend une combinaison d'au moins deux des composés suivants : Apigénine, Berbérine et Monensine.

3. Composition pharmaceutique ou vétérinaire pour son utilisation selon l'une des revendications 1 à 2, **caractérisée en ce qu'**elle comprend en outre au moins un autre agent antiviral.

4. Composition pharmaceutique ou vétérinaire pour son utilisation selon la revendication 3, **caractérisée en ce que** l'autre agent antiviral est choisi parmi les composés suivants : un inhibiteur de protéase, un inhibiteur d'hélicase, et un inhibiteur de l'entrée cellulaire du virus MERS-CoV.

5. Composition pharmaceutique ou vétérinaire pour son utilisation selon la revendication 3 ou 4, **caractérisée en ce que** l'autre agent antiviral est choisi parmi les composés suivants : la ribavirine, un interféron, ou une combinaison des deux.

6. Composition pharmaceutique ou vétérinaire pour son utilisation selon l'une des revendications 1 à 5, **caractérisée en ce qu'**elle est sous une forme galénique destinée à une administration par inhalation.

7. Composition pharmaceutique ou vétérinaire, comprenant dans un véhicule pharmaceutique approprié, une combinaison de berbérine et de monensine.

8. Composition pharmaceutique ou vétérinaire, comprenant dans un véhicule pharmaceutique approprié, au moins une combinaison d'un interféron, de monensine, d'apigénine et de berbérine.

9. Composition pharmaceutique ou vétérinaire selon l'une des revendications 7 ou 8, **caractérisée en ce qu'**elle est sous une forme galénique destinée à une administration par inhalation.

## Patentansprüche

1. Pharmazeutische oder tierarzneiliche Zusammensetzung für ihre Verwendung zur Vorbeugung und/oder Behandlung einer Infektion mit dem Coronavirus MERS-CoV (Middle-East Respiratory Syndrome), **dadurch gekennzeichnet, dass** sie in einem geeigneten pharmazeutischen Träger mindestens eine Verbindung umfasst, die aus dem Apigenin und dem Berberin ausgewählt ist.

2. Pharmazeutische oder tierarzneiliche Zusammensetzung für ihre Verwendung nach Anspruch 1, **dadurch gekennzeichnet, dass** sie eine Kombination von mindestens zwei der folgenden Verbindungen umfasst: Apigenin, Berberin und Monensin.

3. Pharmazeutische oder tierarzneiliche Zusammensetzung für ihre Verwendung nach einem der Ansprüche 1 bis 2, **dadurch gekennzeichnet, dass** sie ferner mindestens ein anderes antivirales Mittel umfasst.

4. Pharmazeutische oder tierarzneiliche Zusammensetzung für ihre Verwendung nach Anspruch 3, **dadurch gekennzeichnet, dass** das andere antivirale Mittel aus den folgenden Verbindungen ausgewählt ist: einem Proteasehemmer, einem Helicasehemmer und einen Zelleintrittshemmer des Virus MERS-CoV.

5. Pharmazeutische oder tierarzneiliche Zusammensetzung für ihre Verwendung nach Anspruch 3 oder 4, **dadurch gekennzeichnet, dass** das andere antivirale Mittel aus den folgenden Verbindungen ausgewählt ist: dem Ribavirin, einem Interferon oder einer Kombination der beiden.

6. Pharmazeutische oder tierarzneiliche Zusammensetzung für ihre Verwendung nach einem der Ansprüche 1 bis 5, **dadurch gekennzeichnet, dass** sie in einer galenischen Form vorliegt, die für eine Verabreichung durch Inhalation bestimmt ist.

7. Pharmazeutische oder tierarzneiliche Zusammensetzung, umfassend in einem geeigneten pharmazeutischen Träger eine Kombination aus Berberin und Monensin.

8. Pharmazeutische oder tierarzneiliche Zusammensetzung, umfassend in einem geeigneten pharmazeutischen Träger mindestens eine Kombination aus einem Interferon, Monensin, Apigenin und Berberin.

9. Pharmazeutische oder tierarzneiliche Zusammensetzung nach einem der Ansprüche 7 oder 8, **dadurch gekennzeichnet, dass** sie in einer galenischen Form vorliegt, die für eine Verabreichung durch Inhalation bestimmt ist.

## Claims

1. A pharmaceutical or veterinary composition for its use in preventing and/or treating a MERS-CoV (Middle-East Respiratory Syndrome) coronavirus infection, **characterised in that** it comprises, in a suitable pharmaceutical vehicle, at least one compound chosen from Apigenin and Berberine.

2. The pharmaceutical or veterinary composition for its use according to claim 1, **characterised in that** it comprises a combination of at least two of the following compounds: Apigenin, Berberine and Monensin.

3. The pharmaceutical or veterinary composition for its use according to one of claims 1 to 2, **characterised in that** it further comprises at least another antiviral agent.

4. The pharmaceutical or veterinary composition for its use according to claim 3, **characterised in that** the other antiviral agent is chosen from the following compounds: a protease inhibitor, a helicase inhibitor, and a MERS-CoV virus cell entry inhibitor.

5. The pharmaceutical or veterinary composition for its use according to claim 3 or 4, **characterised in that** the other antiviral agent is chosen from the following compounds: ribavirin, an interferon, or a combination of both.

6. The pharmaceutical or veterinary composition for its use according to one of claims 1 to 5, **characterised in that** it is in a dosage form intended for an administration by inhalation.

7. The pharmaceutical or veterinary composition, comprising in a suitable pharmaceutical vehicle, a combination of berberine and monensin.

8. The pharmaceutical or veterinary composition, comprising in a suitable pharmaceutical vehicle, at least one combination of an interferon, monensin, apigenin and berberine.

9. The pharmaceutical or veterinary composition according to one of claims 7 or 8, **characterised in that** it is in a dosage form intended for an administration by inhalation.
